(19) Europäisches Patentamt
European Patent Office
Office européen des brevets

(11) Publication number: **0 276 096 A1**

# (12) EUROPEAN PATENT APPLICATION

(21) Application number: 88300318.8

(22) Date of filing: 15.01.88

(51) Int. Cl.[4]: C 07 C 2/00
C 07 C 11/02, C 07 C 9/22,
C 07 C 6/04, C 08 F 110/14,
C 07 C 2/12

(30) Priority: 21.01.87 US 5817

(43) Date of publication of application:
27.07.88 Bulletin 88/30

(84) Designated Contracting States:
BE DE FR GB IT NL

(71) Applicant: MOBIL OIL CORPORATION
150 East 42nd Street
New York New York 10017 (US)

(72) Inventor: Absil, Robert Peter
P-311 Heather Ridge Apartments
Mantua New Jersey 08051 (US)

Angevine, Philip Jay
10 Kent Court
West Deptford New Jersey 08051 (US)

Garwood, William Everett
125 Warwick Road
Haddonfield New Jersey 08033 (US)

Quann, Richard John
10 West Spruce Street
Morrestown New Jersey 08057 (US)

(74) Representative: Colmer, Stephen Gary
Patent Department c/o Mobil Services Company Limited
Mobil Court 3 Clements Inn
London WC2A 2EB (GB)

## (54) An olefin metathesis process.

(57) A process for olefin metathesis by oligomerizing lower olefins over medium pore zeolite catalyst to higher olefins then adding an alpha-olefin in the presence of a metathesis catalyst having a low acidity to provide a mixture of alpha-olefins. Metathesis olefins may be polymerized to make a lube product.

EP 0 276 096 A1

**Description**

AN OLEFIN METATHESIS PROCESS

This invention is directed to the manufacture of lubricating oils and lubes.

In the process for catalytic conversion of olefins to heavier hydrocarbons by catalytic oligomerization using a medium pore shape selective acid crystalline zeolite such as ZSM-5 catalyst, process conditions can be varied to favor the formation of hydrocarbons of varying molecular weight. At moderate temperature and relatively high pressure, the conversion conditions favor C10+ aliphatic product. Lower olefinic feedstocks containing $C_2$-$C_8$ alkenes may be converted; however, the distillate mode conditions do not convert a major function of ethylene. U.S. Patent No. 4,547,612 discloses a continuous process for the catalyst conversion of olefins to lubricant or heavy distillate range compounds in which a light olefin feedstock, e.g., propylene, is combined with a $C_5+$ olefin stream recovered from previous product effluent.

Olefin metathesis (disproportionation) is a known type of reaction in which one or more olefinic compounds are transformed into other olefins of different molecular weights. The reaction of an olefin with itself to produce an olefin of a higher molecular weight and an olefin of a lower molecular weight can also be referred to as a self-disproportionation. For example, propylene can be disproportionated to ethylene and cis-, and trans-2-butene. Another type of disproportionation involves the cross-disporoportionation of two different olefins to form still other olefins. An example would be the reaction of one molecule of 2-butene with one molecule of 3-hexane to produce two molecules of 2-pentene. Another example of a cross-disproportionation involves the reaction of an internally unsaturated olefin with an alpha-olefin to provide two different alpha-olefins, e.g., the reaction of 2,4,4-trimethyl-2-pentene with ethylene to provide equimolar amounts of 3,3-dimethyl-1-butene (neohexene) and isobutene as shown in Banks, "Olefin Metathesis: Technology and Application", Applied Industrial Catalysis, Vol. 3, Chapter 7, pp. 215 et seq. Leach, ed. (1984).

Among the catalysts that have been developed for olefin metathesis are those comprising inorganic refractory materials containing a catalytic amount of at least one of molybdenum oxide and tungsten oxide on silica. Other olefin metathesis processes and catalyst compositions therefor are described in U.S. Patent Nos. 3,883,606; 3,915,897; 3,952,070; 4,180,524; 4,317,948; 4,409,409; 4,431,855; 4,499,328; 4,504,694; 4,517,401; 4,547,617; and 4,609,769 among others.

The terms "disproportionation" and "metathesis" as used herein mean the conversion of an olefinic hydrocarbon feed to a mixture of product olefinic hydrocarbons having different numbers of carbon atoms than the feed olefins.

The present invention provided a process for olefin metathesis which comprises oligomerizing one or more lower olefins over a medium pore zeolite to higher olefins and reacting these with added alpha olefins in the presence of a shape selective zeolite olefin metathesis catalyst to form metathesized alpha olefins characterized by use of metathesis catalyst having an alpha value less than 100.

The initial operation in which a light olefin feed is converted to higher olefin can take place in a single reactor or a series of reactors. In the case of the latter, each reactor can be packed with the same or a different zeolite catalyst. Essentially any of the known zeolite-catalyzed olefin oligomerization processes can be employed in carrying out the first step of the lube production process herein.

The shape-selective oligomerization/polymerization catalysts are known. Preferred catalysts for use in the oligomerization operation of the process herein include the medium pore crystalline metallosilicate zeolites having a metal (typically silica) to alumina molar ratio of at least 12, a constraint index of from 1 to 12 and acid cracking activity of 50-300. Representative of the ZSM-5 type zeolites are ZSM-5, ZSM-11, ZSM-12, ZSM-23, ZSM-35 and ZSM-38. ZSM-5 is disclosed and claimed in U.S. Patent No. 3,702,886 and U.S. Patent No. Re. 29,948; ZSM-11 is disclosed and claimed in U.S. Patent No. 3,709,979. Also, see U.S. Patent No. 3,832,449 for ZSM-12; U.S. Patent No. 4,076,842 for ZSM-23; U.S. Patent No. 4,016,245 for ZSM-35 and U.S. Patent No. 4,046,839 for ZSM-38. A suitable shape selective medium pore catalyst for fixed bed use is a small crystal HZSM-5zeolite (silica-alumina ratio of 70:1) with alumina binder in the form of cylindrical extrudates of 1-5mm. Unless otherwise stated in this description, the catalyst shall consist essentially of ZSM-5 which has a crystallite size of 0.02 to 0.05 micron. Other pentasil catalysts which can be used in one or more reactor stages include a variety of medium pore (5 to 9 angstroms) siliceous materials such as borosilicates, ferrosilicates, and/or aluminosilicates such as are disclosed in U.S. Patent Nos. 4,414,423 and 4,417,088.

The surface activity of these and similar catalysts can be modified by pretreatment, e.g., with a surface-neutralizing base as disclosed in U.S. Patent No. 4,520,221.

Shape-selective oligomerization as it applies to the conversion of C2-C10 olefins over ZSM-5 is known to produce higher olefins of up to C30 and even higher. As reported by Garwood in "Conversion of C2-C10 to Higher Olefins over Synthetic Zeolite ZSM-5," ACS Symposium Series, No. 218, Intrazeolite Chemistry (American Chemical Society 1983), reaction conditions favoring higher molecular weight product are lower temperature (200-260°C), elevated pressure (2000 kPa or greater), and long contact time (less than 1 WHSV). The reaction under these conditions proceeds through the acid-catalyzed steps of (1) oligomerization, (2) isomerization-cracking to a mixture of intermediate carbon number olefins, and (3) interpolymerization to give a continuous boiling product containing all carbon numbers. The channel systems of ZSM-5 type catalysts impose shape-selective constraints on the configuration of the large molecules, accounting for the differences with other catalysts.

Conventional temperatures, pressure and equipment can be used for oligomerization. Preferred temperatures can vary from 100 to 350°C, more preferably from 150° to 250°C, with pressures varying from atmospheric to 20,000 kPa (3000 psig) and a WHSV of 0.01 to 2.0 preferably 0.2 to 1.0.

The oligomerizate will contain a complex mixture of higher olefins the specific nature of which will depend upon the type of feed stock utilized and the oligomerization conditions employed. For example, conversion of the aforesaid FCC off-gas containing nearly 65 weight percent combined ethylene and propylene under conditions favoring the production of gasoline and distillate range olefins may provide a mixture of higher olefins within the carbon number range 9-20. Conversion conditions calculated to maximize the production of distillate and lube range producte will provide a higher carbon number range, e.g., 20 to 30 and even higher. Any of these mixtures of higher olefins can be employed as feed stock for the subsequent metathesis operation without further processing. However, if desired, the higher olefin mixtures can be separated by fractionation into two or more fractions with only a particular fraction being subjected to metathesis in accordance with this invention.

The metathesis (disproportionation) conversion of the olefinic hydrocarbons resulting from the olefin oligomerization operation are converted to alpha olefins in a primary reaction which can be thought of as comprising the breaking of two unsaturated bonds between first and second carbon atoms and between third and forth carbon atoms, respectively, and the formation of two new alpha olefinic bonds in different molecules as in the following formulas (illustrating ethylene as the feed alpha-olefin):

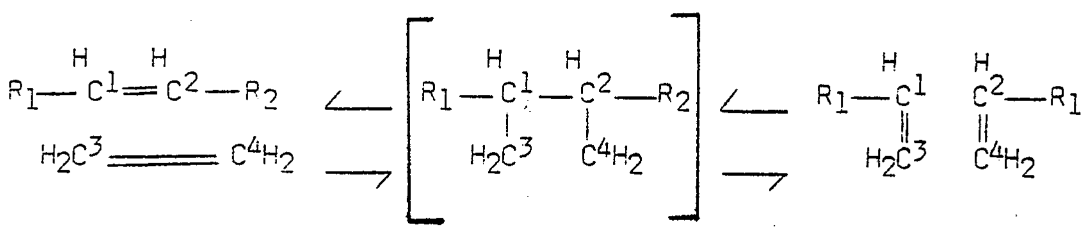

In general any of the C2-8 alpha olefins can be reacted with the oligomerization product effluent in the metathesis operation herein. Some specific examples of such alpha-olefins are ethylene, propylene, 1-butene, 1-pentene, 1-hexane, 1-octene, and the like, with ethylene being preferred.

Any of the catalysts heretofore employed in olefin metathesis are suitably utilized in the second stage conversion herein. Many of these catalysts have been reported in the prior art. Thus, for example, the disporoportionation catalyst can be an oxide of molybdenum, tungsten, or rhenium deposited on a support of silica, alumina, silica-alumina or aluminum phosphate. An additional metal oxide, e.g., a rate earth metal oxide, can also be present as is known. Prior to its use, the catalyst is activated by calcination carried out in a conventional manner. One such catalyst which has been found to provide generally good results is molybdenum oxide supported on a mixture of amorphous precipitated silica and colloidal silica.

In a preferred embodiment of the present invention, a medium pore crystalline silicate zeolite catalyst such as any of those previously mentioned but which is substantially free of Bronsted acid activity is employed as the metathesis catalyst. The use of such a catalyst in metathesis is believed to be unique to the present invention.

The acid activity of the useful medium pore silicate zeolite metathesis catalysts can be expressed in terms of alpha value which reflects the relative activity of the catalyst with respect to a high activity silica-alumina cracking catalyst.

The medium pore crystalline silicate zeolite metathesis catalyst herein will posses an alpha value less than 100, preferably less than 30 and still more preferably less than 10. Thus, for example, a silica-bound HZSM-5 catalyst having an alpha value of 2 provides entirely acceptable results. The presence of at least one metal oxide selected from the group consisting of tungsten oxide, molybdenum oxide rhenium oxide applied to the zeolite by any of the known methods has been observed to increase the yield of lower olefins, particularly butenes and pentenes. The metal oxide can be present in an amount which is typical of known metathesis catalysts.

Preferred conditions for the metathesis reaction include a pressure atmospheric to 35000 kPa (0-5000 psig) a temperature of ambient to 538°C (1000°F), and space velocities of 1 to 300 WHSV based on the nature of the metathesis catalyst. Although the activity of the catalyst is suitable within the broad ranges mentioned above, increased activity is generally found when the pressure is 800 to 3500 kPa (100 to 500 psig), the temperature range is 343 to 454°C (650°-850°F), and the WHSV is from 0.5 to 1000. The particular temperature, pressure and flow rates utilized within these ranges is largely dependent on the properties of the feed material undergoing the metathesis conversion. The process can be carried out with an optional diluent. Diluents comprising paraffinic and cycloparaffinic hydrocarbons can be employed. Suitable diluents are, for example, propane, cyclohexanes, methylcyclohexane, normal pentane, normal hexane, iso-octane, dodecane, and the like, or mixtures thereof, including primarily those paraffins and cycloparaffins having up to 12 carbon atoms per molecule. The diluent should be nonreactive under the conditions of the reaction. In some instances the

use of the diluent can increase the selectivity of the conversion to primary products. The reaction can also be carried out in a single unit or a battery of units employing the same or a different catalyst.

The amount of alpha-olefin employed in the metathesis conversion can vary widely and will depend in part on the degree of unsaturation in the higher olefin feed which can be readily quantified employing known techniques, e.g., bromine number. Generally, the alpha-olefin will be present in stoichiometric excess of the amount theoretically required by can be substantially less than this. If desired, excess alpha-olefin can be separated from the metathesis product effluent and recycled.

It is within the scope of the present application to separate the mixture of alpha olefins resulting from the disproportionation operation into at least two fractions, e.g., a fraction made up of lighter products such as those in the C3 to C8 range and a fraction largely containing C9+ products, and to recycle the lighter fraction as cofeed for the initial oligomerization reaction.

All or a part of the alpha-olefins obtained from the metathesis step are converted to lube range products by further oligomerization/interpolymerization, collectively referred to herein as "polymerization", over a suitable catalyst or free radical initiator. Thus, the following can be used: acid catalysts such as silica-alumina and crystalline silicate zeolites such as ZSM-5; Fridel-Crafts catalysts such as aluminum chloride and boron trifluoride; anionic catalysts such as lithium and sodium alkyls and lithium and sodium naphthalenes; polymerization catalysts of the Ziegler-Natta type; and free radical initiators such as the organic peroxides and hydroperoxides. Temperatures of 100 to 260°C generally provide good results.

Ordinarily, it is desirable to subject the polymerizate to a conventional hydrogenation procedure to effect its stabilization. This can be readily accomplished using any conventional hydrogenation catalyst such as nickel on kieselguhr at temperatures ranging from 149 to 427°C (300 to 800°F) and at pressures ranging from 800 to 7000 kPa (100 to 1000 psig).

The following examples are further illustrative of the invention.

EXAMPLE 1

This example illustrates the preparation of a metathesis catalyst composition made up of molybdenum oxide on a mixture of amorphous precipitated silica and colloidal silica.

75 wt.% amorphous precipitated silica (HiSil 233 EP of PPG Industries) and 25 wt.% colloidal silica (Ludox Hs-30 of DuPont) were mulled and extruded to 1.6 mm (1/16") diameter and the extrudate was dried at 121°C (250°F). Following calcination for (3 hrs. at 538°C (1000°F) in 5v/v/min. air with heating at the rate of 1.7°C/min. (3°F.min.), the extrudate was exchanged twice at ambient temperature in circulating 1 N NH4NO3 (5 ml/gm catalyst), dried at 121°C (250°F) and calcined for 3 hrs. at 538°C (1000°F) in 3v/v/min. air with heating at the rate of 2.8°C.min (5°F/min)/ the extrudate was then impregnated to incipient wetness with an ammonium heptamolybdate solution, dried at ambient temperature for 4 hours, dried at 121°C (250°F) for 14 hours and calcined for 3 hrs. at 538°C (1000°F) in static air with heating at the rate of 2.8°C/min (5°F/min). the molybdenum content of the final catalyst was 3.7 wt.%.

EXAMPLE 2

This example is illustrative of the lube production process of the present invention employing the metathesis catalyst of Example 1.

(A) Olefin Oligomerization To Provide Higher Internal Olefins

Fluidized catalytic cracking (FCC) off-gases consisting of 42.5 wt% C3=, 14.5 wt.% C3, 22.1 wt.% C4= and 3.3 wt.% C4 were processed over HZSM-5 at 4200 kPa (600 psig), 0.6 WHSV based on olefin charge, 2/1 gas and 1/1 liquid recycled ratio and 204-260°C (400-500°F) giving 80% conversion of olefin to liquid. The gasoline portion of the liquid was stripped off and the bottoms were analyzed as follows:

| | | |
|---|---|---|
| Gravity, °API | 49.5 | |
| g/cc | 0.788 | |
| Dist., (ASTM D-2887) | | |
| | °F | °C |
| 1 % | 255 | 124 |
| 5 % | 305 | 152 |
| 10% | 320 | 160 |
| 30% | 363 | 184 |
| 50% | 412 | 211 |
| 70% | 463 | 239 |
| 90% | 567 | 297 |
| 95% | 629 | 332 |
| 99% | 762 | 406 |
| Mol. Wt., (Calculated) | 185 | |
| Bromine No. | 95.5 | |

The properties of the bottoms indicated essentially 100% olefins, 9-20 carbon numbers and an average carbon number of 13.

(B) Olefin Metathesis

The catalyst of Example 1 was sized to 0.71-1.4 mm (14-25 mesh) and 5.0 g (10.4 cc) placed in a 9.5 mm (3/8") internal diameter stainless steel reactor. The liquid from olefin oligomerization stage (A) and ethylene (Matheson, 99.5% min. purity) were then cofed over the catalyst at 2900 kPa (400 psig) and at the rates and temperatures listed below:

| | | Olefin Metathesis Conditions | | | | | |
|---|---|---|---|---|---|---|---|
| Olefinic Liquid from stage (A), WHSV | | --- | 0.8 | --- | --- | 1.6 | --- |
| Ethylene, WHSV | | --- | 0.4 | --- | --- | 0.4 | --- |
| Mol Ratio, C2=/Liq. Olefin | | --- | 10 | --- | --- | 5 | |
| Temp., °F | | 701 | 750 | 803 | 752 | 801 | 849 |
| °C | | 372 | 399 | 428 | 400 | 427 | 454 |
| Ethylene Conv., wt.% | | 6 | 23 | 58 | 17 | 51 | 77 |
| Yields, wt.% (excluding unreacted ethylene) | Charge to the Reactor | | | | | | |
| C1-C4 | | 1.5 | 4.9 | 5.0 | 2.8 | 7.2 | 11.9 |
| C5+ | | 98.5 | 95.1 | 95.0 | 97.2 | 92.8 | 88.1 |
| C5 Olefins | | 0.2 | 1.0 | 2.2 | 0.7 | 1.9 | 3.4 |
| Liq. B.P., (ASTM D-2887) | | | | | | | |
| 10% °F | 320 | 314 | 268 | 195 | 301 | 229 | 158 |
| °C | 160 | 157 | 131 | 91 | 149 | 109 | 70 |
| 99% °F | 762 | 754 | 724 | 701 | 735 | 722 | 732 |
| °C | 406 | 401 | 384 | 372 | 391 | 383 | 389 |
| | | Olefin Composition | | | | | |
| 1-pentene | | 12 | 18 | 16 | 17 | 21 | 24 |
| 2-methyl-1-butene | | 24 | 27 | 25 | 34 | 32 | 31 |
| 3-methyl-1-butene | | 2 | 3 | 3 | 3 | 4 | 4 |
| trans-2-pentene | | 9 | 12 | 11 | 10 | 12 | 13 |
| cis-2-butene | | 4 | 4 | 5 | 4 | 6 | 6 |
| 2-methyl-2-butene | | 49 | 36 | 40 | 32 | 25 | 22 |
| Time on Stream, Days | | 2.3 | 3.2 | 4.3 | 6.0 | 7.7 | 8.6 |

Theoretical ethylene conversions of 10% and 20% for the 10/1 and 5/1 mole ratios were exceeded at the higher temperatures indicating thermal and/or catalytic reactions in addition to metathesis had taken place.

(C) Polymerization of Alpha-olefins Resulting From Olefin Metathesis

Polymerizations were carried out by charging 100 g liquid to a 500 ml round bottom glass flask, equipped with stirrer, condenser plus take-off, thermometer and dropping funnel. The liquid charge was heated to 150°C, 20g (26 ml) of ditertiary butyl peroxide was added dropwise over a one hour period, the temperature was held at 150°C for another 3 hours and then increased to 180°C in 2 hours. The results were as follows:

| Charge to Polymerization | Oligomerization Effluent Polymerization Product | Metathesis Effluent Polymerization Product | |
|---|---|---|---|
| $C_2$=/Liquid Olefin Ratio | - | 10 | 5 |
| Temp., °F | - | 750 | 849 |
| °C | | 399 | 454 |
| 343°C+ (650°F+) Product | | | |
| (ASTM D-2887), wt.% | 28 | 28 | 29 |
| Lube Yield, wt.% | 33 | 32 | 33 |
| Gravity, °API | 35.4 | 33.5 | 28.3 |
| g/cc | 0.847 | 0.857 | 0.885 |
| Pour Point, °F | -65 | -60 | -45 |
| °C | -54 | -51 | -43 |
| K.V. @40°C, cs | 28.90 | 35.02 | 68.68 |
| K.V. @100°C, cs | 4.74 | 5.27 | 7.39 |
| SUS @100°F (38°C) | 150 | 182 | 361 |
| Viscosity Index | 70.4 | 71.8 | 57.6 |
| 95% Boiling Point °F | 977 | 1003 | 1045 |
| °C | 525 | 539 | 563 |

As these data show, the 343°C+ (650°F+) product yields from polymerization of the oligomerization product and polymerization of the metathesis product were essentially the same but the viscosities of the lubes from the latter were significantly higher.

EXAMPLE 3

This example illustrates the preparation of a preferred metathesis catalyst composition made up of silica-bound ZSM-5 (alpha value of about 2).

65 wt.% ASM-5 (silica to alumina ratio of 510) was combined with a binder mixture of 17.5 wt.% colloidal silica (Ludox HS-30) and 17.5 wt.% amorphous precipitated silica (HiSil233 EP). The mixtures was mulled, extruded to 1/6 mm (1.16") diameter and the extrudate was dried at 121°C (250°F). A first calcination carried out for 3 hrs. at 538°C (1000°F) in 5 v/v/min. nitrogen with heating at the rate of 1.7°C/min (3°F/min) was followed by a second 3hr. period of calcination at 538°C (1000°F) in 5v/v/min. air after which the extrudate was steamed at 538°C (1000°F) for 10 hrs. in 1 atm. steam with heating at a rate of 2.8°C/min (5°F/min). The extrudate was then exchanged twice at ambient temperature in circulating 1 N NaNO3 (5ml/gm catalyst), dried at 121°C (250°F) and calcined for 3 hrs. at 538°C (1000°F) in 3 v/v/min. air with heating at the rate of 2.8°C/min (5°F/min). Following this operation, the extrudate was again exchanged twice at ambient temperaure for 1 hr. with circulating 1 M Ca(NO3).6H20 (5 ml/gm catalyst), dried at 121°C (250°F) and finally calcined for 3 hrs. at 538°C (1000°F) in 5 v/v/min. air with heating at the rate of 2.8°C/min (5°F/min) to provide a silica-bound HZSM-5 catalyst of approximately 2 alpha value.

EXAMPLE 4

This example is illustrative of the lube protection process of the present invention employing the metathesis catalyst of Example 3 and olefinic product (bottoms) from oligomerization stage (A) of Example 2 as feed for the metathesis stage.

(A) Olefin Metathesis

The silica-bound HZSM-5 catalyst of Example 3 was sized to 0.71 to 1.4 mm (14-25 mesh) and 5.0 g (10.4 cc) placed in a 9.5 mm (3/8") internal diameter stainless steel reactor. Olefinic product from step (A) of Example 2 and ethylene (Matheson, 99.5% min. purity) were then cofed over the catalyst at 2900 kPa (400 psig), the liquid at 1.5 WHSV and ethylene at 0.4-0.5 WHSV, at the temperatures indicated below:

| | | | | |
|---|---|---|---|---|
| Temp., °F | 600 | 699 | 803 | 850 |
| °C | 316 | 371 | 428 | 454 |
| Ethylene Conv., wt% | 3 | 16 | 61 | 83 |
| Yields, wt% (excluding unreacted ethylene) | | | | |
| $C_1$-$C_4$ | 0.1 | 0.4 | 6.3 | 13.0 |
| $C_5$+ | 99.9 | 99.6 | 93.7 | 87.0 |
| Propylene | - | 0.2 | 0.5 | 0.9 |
| Butenes | 0.1 | 0.1 | 2.8 | 4.5 |
| Pentenes | 0.1 | 0.1 | 2.1 | 2.7 |
| C5 Olefin Composition | | | | |
| 1-pentene | - | - | 12 | 13 |
| 2-methyl-1-butene | - | - | 18 | 17 |
| 3-methyl-1-butene | - | - | 2 | 3 |
| trans-2-pentene | - | - | 19 | 19 |
| cis-2-butene | - | - | 9 | 10 |
| 2-methyl-2-butene | - | - | 40 | 38 |

The liquid product obtained at 428°C (803°F) had the following boiling range:

| wt. % | Temp. °F | Temp °C |
|---|---|---|
| 1 | 35 | 2 |
| 10 | 212 | 100 |
| 30 | 335 | 168 |
| 50 | 384 | 196 |
| 70 | 444 | 229 |
| 90 | 546 | 286 |
| 95 | 609 | 321 |
| 99 | 738 | 392 |

(B) Polymerization of Alpha-olefins resulting from Olefin Metathesis

This liquid product was topped to remove an approximately 10 wt% overhead, boiling below the range of the liquid charge to metathesis. The bottomes, 0.786 g/cc (48.9° API gravity), and olefinic product from step (A) of Example 2 were polymerized by charging 100g liquid to a 500 ml round bottom glass flask equipped with stirrer, condenser plus take-off, thermometer and droppinglfunnel. The liquid charge was heated to 150° C, 20g (20 ml) of ditertiary butyl peroxide was added dropwise over a one-hour period and the temperature was held at 150°C for another 3 hours and then increased to 180°C in 2 hours. The results were as follows:

| | Product Obtained from Polymerization of Oligomerizate of Example-2(A)----- | Product Obtained from Polymerization of Metathesis Product of-Example-4(A)--- |
|---|---|---|
| Lube Yield, wt % | 33 | 37 |
| Gravity, °API | 35.4 | 32.8 |
| Specific | 0.8478 | 0.8612 |
| Pour Point, °F | -65 | -45 |
| °C | -54 | -43 |
| K.V. at 40°C, cs. | 28.90 | 34.08 |
| K.V. at 100, c.s. | 4.74 | 5.22 |
| SUS at 100°F (38°C) | 150 | 177 |
| Viscosity Index | 70.4 | 74.6 |

As these data show, the lube derived from the metathesis liquid has both a higher viscosity and higher viscosity index than that derived from the original oligomerizate.

EXAMPLE 5

This example illustrates the effect of tungsten oxide on the metathesis catalyst of Example 3.

Tungsten was placed on the metathesis catalyst of Example 3 by impregnating to incipient wetness with ammonium metatungstate solution, drying at room temperature for 4 hours, then 121°C (250°F) overnight, and finally calcining at 538°C (1000°F) for 3 hours in static air. The tungsten content (present as tungsten oxide) of the final catalyst was 6.8 wt.%. The olefinic liquid of Example 2(A) and ethylene were cofed over this catalyst as described in Example 4(A), with the following results:

| | | | | |
|---|---|---|---|---|
| Temp., °F | 600 | 703 | 801 | 851 |
| °C | 316 | 373 | 427 | 455 |
| Ethylene Conv., wt % | 2 | 13 | 67 | 86 |
| Yields, NLB (excluding unreacted ethylene) | | | | |
| $C_1$-$C_4$ | 0.6 | 4.4 | 13.3 | 20.0 |
| $C_5$+ | 99.4 | 95.6 | 86.7 | 80.9 |
| Propylene | 0.3 | 0.9 | 0.9 | 0.7 |
| Butenes | 0.3 | 3.3 | 9.5 | 12.0 |
| Pentenes | 0.3 | 2.6 | 9.0 | 7.4 |
| C5 Olefin Composition: | | | | |
| 1-pentene | - | 7 | 5 | 6 |
| 2-methyl-1-butene | - | 14 | 19 | 19 |
| 3-methyl-1-butene | - | 2 | 3 | 3 |
| trans-2-pentene | - | 22 | 12 | 14 |
| cis-2-butene | - | 11 | 9 | 8 |
| 2-methyl-2-butene | - | 44 | 52 | 50 |

These data show that the effect of the tungsten oxide, compared to the catalyst of Example 3(B) which contains no tungsten oxide, is to increase the yield of $C_3$-$C_5$ olefins but with fewer 1-olefins in the pentene product.

EXAMPLE 6

This example illustrates the effect of a hydrogen pretreatment on the metathesis catalyst of Example 5.

A fresh portion of the catalyst was reduced in situ with hydrogen at 482°C (900°F) and at atmospheric pressure for 64 hours and the olefinic liquid and ethylene cofed, as described in Example 4(A), with the following results:

| | | | | |
|---|---|---|---|---|
| Temp., °F | 601 | 702 | 801 | 853 |
| °C | 316 | 372 | 427 | 456 |
| Ethylene Conv., wt % | 1 | 9 | 53 | 79 |
| Yields, (excluding unreacted ethylene) | | | | |
| $C_1$-$C_4$ | 0.4 | 0.9 | 7.9 | 14.9 |
| $C_5$+ | 99.6 | 99.1 | 92.1 | 85.1 |
| Propylene | - | 0.3 | 0.8 | 1.6 |
| Butenes | - | 0.3 | 4.4 | 5.7 |
| Pentenes | - | 0.3 | 3.1 | 4.0 |
| C5 Olefin Composition | | | | |
| 1-pentene | - | - | 11 | 20 |
| 2-methyl-1-butene | - | - | 22 | 16 |
| 3-methyl-1-butene | - | - | 2 | 2 |
| trans-2-pentene | - | - | 22 | 17 |
| cis-2-butene | - | - | 1 | 10 |
| 2-methyl-2-butene | - | - | 43 | 35 |

The effect of the hydrogen pretreatment was a small decrease in ethylene conversion, a large decrease in $C_4$ and $C_5$ olefin production but a significant increase in the 1-olefin content of the pentene fraction.

EXAMPLE 7

This example illustrates the effect of incorporating twice the amount of tungsten in the metathesis catalyst of Example 3.

Tungsten (as tungstyen oxide) was placed on the catalyst of Example 3 in substantially the same manner as described in Example 5. The tungsten content of the final catalyst was 14.5 wt % (as compared to 6.8 wt % for the catalyst of Example 3). The olefinic liquid of Example 2(A) and ethylene were cofed over this catalyst as described in Example 4(A) with the following results:

| | | | | |
|---|---|---|---|---|
| Temp., °F | 601 | 700 | 802 | 850 |
| °C | 316 | 371 | 428 | 454 |
| Ethylene Conv., wt % | 5 | 13 | 96 | 98 |
| Yields, (excluding unreacted ethylene) | | | | |
| $C_1$-$C_4$ | 0.7 | 2.0 | 11.9 | 17.7 |
| $C_5$+ | 99.3 | 98.0 | 88.1 | 82.3 |
| Propylene | 0.1 | 0.7 | 0.4 | 0.6 |
| Butenes | 0.2 | 0.8 | 6.4 | 5.6 |
| Pentenes | 0.2 | 2.3 | 7.1 | 5.4 |
| C5 Olefin Composition | | | | |
| 1-pentene | - | 6 | 4 | 5 |
| 2-methyl-1-butene | - | 14 | 18 | 18 |
| 3-methyl-1-butene | - | 3 | 3 | 2 |
| trans-2-pentene | - | 21 | 14 | 15 |
| cis-2-butene | - | 12 | 8 | 8 |
| 2-methyl-2-butene | - | 44 | 53 | 52 |

The effect of doubling the tungsten content from 6.8 to 14.5 wt% was to increase ethylene conversion but to decrease the yield of C4 and C5 olefins. The liquid product from the 428°C (802°F) run had the following boiling range:

| Wt.-% | Temp.-°F | °C |
|---|---|---|
| 1 | -21 | -29 |
| 10 | 96 | 36 |
| 30 | 208 | 98 |
| 50 | 319 | 159 |
| 70 | 397 | 203 |
| 90 | 520 | 271 |
| 95 | 588 | 309 |
| 99 | 726 | 386 |

The metathesis product was topped to remove an approximately 35 wt % overhead, boiling below the range of the liquid charge (from Example 2(A)) as in Example 4(B). The bottoms were polymerized to lube using 20 wt% ditertiary butyl peroxide as in Example 4(B) with the following results:

Lube Yield, wt % 36
Gravity, °API 29.9
Gravity, Specific 0.8767
Pour Point, °F -40
Pour Point °C -40
K.V. at 40°C, cs. 43.42
K.V. AT 100°C, cS. 5.75
SUS at 100°F (38°C) 226
Viscosity Index 57.1

Although the lube viscosity is higher than that of Example 4(B), the viscosity index is much lower. The yield based on the original olefinic liquid is only 23% compared to 33 in Example 4(B). Thus, the 2 alpha HZSM-5 without tungsten is preferred for making lubes while the tungsten-containing catalyst is preferred for making olefins below the carbon number of the liquid charge, of varying 1-olefin content.

The foregoing examples demonstrate both the versatility and the unexpected results of the process of this invention employing the preferred low alpha value medium pore crystalline silicate zeolites as metathesis catalysts.

EXAMPLE 8

This example illustrates the process of the present invention employing a silica-bound HZSM-5 catalyst having an alpha value of approximately 12.

The catalyst was prepared as described in Example 3 except that the calcined material without steaming and exchanging with $NaNO_3$ and $Ca(NO_3)_2$ as described therein was impregnated with ammonium metatungstate solution as described in Example 5. The tungsten content of the final catalyst was 14.5 wt. %. The liquid of Example 2(a) and ethylene were cofed over the catalyst as described in Example 4(A) with the following results:

| | | | | |
|---|---|---|---|---|
| Temp., °F | 601 | 703 | 800 | 851 |
| °C | 316 | 373 | 427 | 455 |
| Ethylene Conv., wt % | 1 | 11 | 50 | 75 |
| Yields, NLB (excluding unreacted ethylene) | | | | |
| $C_1$-$C_4$ | 0.4 | 1.0 | 7.0 | 13.8 |
| $C_5$+ | 99.6 | 99.0 | 93.0 | 86.2 |
| Propylene | 0.1 | 0.4 | 0.6 | 1.2 |
| Butenes | 0.1 | 0.4 | 4.1 | 5.8 |
| Pentenes | 0.1 | 0.5 | 2.9 | 3.7 |
| $C_5$ Olefin Composition | | | | |
| 1-pentene | - | - | 9 | 9 |
| 2-methyl-1-butene | - | - | 20 | 18 |
| 3-methyl-1-butene | - | - | 2 | 3 |
| trans-2-pentene | - | - | 19 | 20 |
| cis-2-butene | - | - | 9 | 11 |
| 2-methyl-2-butene | - | - | 41 | 39 |

Compared to the 6.8 wt% tungsten on 2 alpha HZSM-5 of Example 5, the use of the catalyst of this example results in less C4 and C5 olefins but is nevertheless active as a metathesis catalyst.

COMPARATIVE EXAMPLE

This example demonstrates that a medium pore crystalline metallosilicate zeolite having an alpha value much above 100 is unsuitable as a metathesis catalyst.

An HZSM-5 zeolite having a silica to alumina ratio of about 55 and a high solids, low sodium (HSLS) content was mulled with silica binder, extruded, and dried as described for the catalyst of Example 3, then impregnated with ammonium metatungstate as described in Example 5. The tungsten content of the final catalyst was 6.6 wt %. The olefinic liquid of Example 2(A) and ethylene were cofed over the catalyst (alpha value of about 168) using the equipment and procedure of Example 4(A) with the following results:

Temp., °F 594
Temp., °C 312
Ethylene Conv., wt % 95

Yields, NLB (excluding unreacted ethylene)
C1-C4 5.5
C5+ 94.5
Propylene 0.1
Butenes 1.1
Pentenes 1.1

C5 Olefin Composition
1-pentene 3
2-methyl-1-butene 18
3-methyl-1-butene 2
trans-2-pentene 11
cis-2-butene 6
2-methyl-2-butene 60

As these data show, the acidity of the catalyst expressed in terms of apha value is too high to provide acceptable results. While the ethylene conversion is higher, even at 312°C (594°F), there is low conversion to $C_4$ and $C_5$ olefins and a low 1-olefin content in the $C_5$ olefins. The liquid product has a 399° (751°F) boiling point at 95% overhead compared to 335° (635°F) for the charge indicating that considerable olefin polymerization to higher molecular weight hydrocarbons has taken place.

**Claims**

1. A process for olefin metathesis which comprises oligomerizing one or more lower olefins over a medium pore zeolite to higher olefins and reacting these with added alpha olefins in the presence of a

shape selective zeolite olefin metathesis catalyst to form metathesized alpha olefins characteized by use of metathesis catalyst having an alpha value less than 100.

2. A process according to claim 1 wherein the metathesized alpha olefins are polymerized to form a lube product.

3. A process according to claim 2 wherein the lube product is hydrogenated.

4. The process of any preceding claim further characterized in that the metathesis catalyst is a supported oxide of molybdenum, tungsten or rhenium oxide.

5. The process of any preceding claim further characterized in that the metathesis catalyst is a zeolite having a constraint index 1-12 and has little if any Bronsted acid activity.

6. The process of any preceding claim further characterized in that the metathesis catalyst has an alpha value of less than 30.

7. The process of any preceding claim further characterized in that the metathesis catalyst is reduced with $H_2$ before use.

European Patent Office

# EUROPEAN SEARCH REPORT

Application number

EP 88300318.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | EP - A1 - 0 232 617 (MOBIL OIL) * Claims; example 2 * -- | 1-4 | C 07 C 2/00<br>C 07 C 11/02<br>C 07 C 9/22<br>C 07 C 6/04<br>C 08 F 110/14<br>C 07 C 2/12 |
| P,X | EP - A1 - 0 231 651 (MOBIL OIL) * Claims 1,2,5,6; page 6, line 33 - page 7, line 12 * -- | 1,4 | |
| A | US - A - 4 542 249 (REUSSER) * Examples 1,9 * -- | 1,4 | |
| A | US - A - 4 575 575 (DRAKE et al.) * Example 2 * -- | 1,4 | |
| A | US - A - 4 542 247 (CHANG et al.) * Abstract * -- | 1,3 | TECHNICAL FIELDS SEARCHED (Int. Cl.4) |
| A | US - A - 4 547 613 (GARWOOD et al.) * Abstract; claims * -- | 1 | C 07 C 2/00<br>C 07 C 6/00<br>C 07 C 9/00<br>C 07 C 11/00<br>C 08 F |
| A | PATENT ABSTRACTS OF JAPAN, unexamined applications, field C, vol. 10, no. 144, May 27, 1986<br>THE PATENT OFFICE JAPANESE GOVERNMENT<br>page 27 C 349<br>Kokai-no. 61-2 705 (SHINNEN-RIYOUYU KAIHATSU GIJUTSU KENKIYUU KUMIAI) *<br>---- | 1,5 | |

The present search report has been drawn up for all claims

| Place of search | Date of completion of the search | Examiner |
|---|---|---|
| VIENNA | 18-04-1988 | KÖRBER |

**CATEGORY OF CITED DOCUMENTS**

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document